# EUROPEAN PATENT APPLICATION

(11) **EP 4 372 757 A1**
(43) Date of publication of application: **22.05.2024**
(21) Application number: 22290062.3
(22) Date of filing: 21.11.2022
(51) Int. Cl.: G16H 30/40, G16H 50/20, G16H 70/20, G16H 10/60, G16H 50/30

(54) **METHOD AND SYSTEM FOR PROCESSING MEDICAL IMAGES**

(71) Applicant: Koninklijke Philips N.V., 5656 AG Eindhoven (NL)
(72) Inventor: Gessert, Nils Thorben, 5656 AE Eindhoven (NL); Wehle, Simon, 5656 AE Eindhoven (NL); Oliveira, Lucas de Melo, 5656 AE Eindhoven (NL); De Craene, Mathieu, 5656 AE Eindhoven (NL); Waechter-Stehle, Irina, 5656 AE Eindhoven (NL); Sadeghi, Seyedali, 5656 AE Eindhoven (NL); Pezzotti, Nicola, 5656 AE Eindhoven (NL)
(74) Representative: Philips Intellectual Property & Standards

(57) **Abstract**

The present invention relates to computer implemented methods and systems for processing medical image. The methods include receiving a first acquired image of a patient, wherein the first acquired image is a medical image acquired using a medical imaging device. The first acquired image is compared to a plurality of reference images by determining a first plurality of confidence metrics using a first calculation method. The first plurality of confidence metrics for the first acquired image. Each respective confidence metric of the first plurality of confidence metrics indicates how closely the first acquired image matches a respective reference image of the plurality of reference images.

## Description

### FIELD OF THE INVENTION

The present disclosure relates generally to computer implemented methods and systems for processing medical images and, in particular, to computer implemented methods and systems for matching acquired images to reference images.

### BACKGROUND OF THE INVENTION

Several medical imaging procedures involve a user (i.e., a radiologist or clinician) comparing acquired images to reference images. In particular, the procedure may involve the user matching an acquired image to a particular reference image: One clinical application where this is commonly required is stress echocardiography.

During a cardiac stress echo exam, the sonographer will acquire multiple views of the patient's heart, both in rest and stress stages. The goal is to have a comparison of the same cardiac views in rest and stress stages to help identify any clinical abnormalities (e.g., wall motion abnormalities). The identification of wall motion abnormalities is particularly useful in the assessment of ischemic heart disease.

Stress exam protocols are often very difficult to perform for the sonographer. In particular, getting well-matching views of the rest and stress stage(s) can be challenging.

There are different stress exam protocols depending on geographical locations, hospitals, and device manufacturers. A common stress protocol is to have the sonographer acquire every required view in every stress stage in a pre-defined sequential order. This means the sonographer has to acquire each view at a very specific point in time which is very inflexible and often leads to disruptions and/or views being acquired incorrectly. The success of these protocols is dependent on the sonographer's experience and skill. Accordingly, there is a need to provide more flexible alternatives.

There is a need to optimize the time taken to carry out stress exam protocols.

Generally, patients are put into a stress state using exercise. For example, the patient may be asked to exercise for a specific period of time with the difficulty of exercise incrementally increasing until the patient begins to experience clinical symptoms, or abnormal findings are observed on the acquired images by the user. If abnormal findings are identified on the acquired images, the stress protocol will normally be stopped. However, if the abnormal findings are missed by the user (especially less experienced ones), the exam may continue needlessly. The protocol may also be unnecessarily prolonged because the user is unsure whether all of the required images have been acquired. This is an inefficient use of time and may cause the patient unnecessary discomfort. Conversely, the protocol may be terminated too soon - before all of the required images have been collected. This is also sub-optimal. Accordingly, there is a need for improved methods of medical image processing with the aim of addressing these problems.

### SUMMARY OF THE INVENTION

The invention is defined by the claims.

According to examples in accordance with an aspect of the invention, there is provided a computer implemented method of medical imaging comprising:
receiving a first acquired image of a patient, wherein the first acquired image is a medical image acquired using a medical imaging device;
comparing the first acquired image to a plurality of reference images by determining a first plurality of confidence metrics using a first calculation method; and
outputting the first plurality of confidence metrics for the first acquired image,
wherein each respective confidence metric of the first plurality of confidence metrics indicates how closely the first acquired image matches a respective reference image of the plurality of reference images.

Providing a method in which a plurality of confidence metrics that indicate how closely a medical image matches each of a plurality of reference images makes it possible for a user to perform medical imaging protocols in an improved way. In particular, it is possible to perform known imaging protocols that involve matching acquired images to reference images in a more flexible manner. More specifically, this method allows a user to collect an acquired image that corresponds to any reference image rather than the user being constrained to follow a rigid protocol that requires an acquired image corresponding to a specific reference image to be collected in a specific order. This increased flexibility makes it possible to repeat certain acquisitions without invalidating the whole protocol. This added flexibility can reduce image acquisition time and patient discomfort.

The method may further comprise determining each respective confidence metric of the first plurality of confidence metrics by determining the probability that the first acquired image shows the same view of the patient as the respective reference image of the plurality of reference images.

The plurality of confidence metrics may provide the probability of an acquired image matching the same view as each of a plurality of reference views. This is particularly advantageous when performing medical imaging protocols where it is necessary to image the same view of the patient at different times. This is also advantageous in that it allows the operator to prioritize certain views that may be more relevant in view of the condition and history of the patient being examined.

The method may further comprise:
determining whether the first acquired image matches a respective reference image based on the respective confidence metric, and
in response to determining that the first acquired image matches the respective reference image,
labelling the first acquired image as matching the respective reference image.

The plurality of confidence metrics may be used to determine a match between the acquired image and a reference image. This automatic matching process means that the user does not have to manually identify matches, thereby improving the ease with which medical imaging protocols can be performed.

The comparing step of the method may further comprise:
determining a second plurality of confidence metrics using a second calculation method, wherein the second calculation method calculates the confidence metrics in a different way to the first calculation method; and
calculating a plurality of combined confidence metrics, wherein each respective confidence metric of the plurality of combined confidence metrics indicates how closely the first acquired image matches a respective reference image based on the respective confidence metric of the plurality of first confidence metrics and the respective confidence metric of the plurality of second confidence metrics.

The inventors have realized that the comparison between an acquired image and a plurality of reference images can be improved by using more than one source of information. In particular, the inventors have realized that it is possible to provide an improved method of medical image processing by calculating a plurality of combined confidence metrics which are based on a first plurality of confidence metrics and a second plurality of confidence metrics, where the first and second plurality of confidence metrics have been calculated in different ways.

In embodiments involving determining a second plurality of confidence metrics, the method may further comprise determining each respective confidence metric of the second plurality of confidence metrics by:
determining the degree of similarity between the first acquired image and the respective reference image of the plurality of reference images, or
determining the correspondence between anatomical features detected in the first acquired image and the respective reference image.

Multiple different types of information can be extracted from medical images. The inventors have realized that the comparison between an acquired image and a plurality of reference images can be improved by using information relating to the similarity between an acquired image and a plurality of reference images or the correspondence between the anatomical features detected in the images being compared. Using these means of determining confidence metrics can help to provide a more reliable comparison between images. The degree of similarity between images may be determined using a similarity model obtained by a contrastive learning strategy (i.e. a contrastive similarity model).

In embodiments involving determining a second plurality of confidence metrics, the comparing step may further comprise determining a third plurality of confidence metrics using a third calculation method, wherein the third calculation method calculates the confidence metrics in a different way compared to first and second calculation methods, and
calculating a plurality of combined confidence metrics, wherein each respective combined confidence metric indicates how closely the first acquired image matches a respective reference image based on the respective confidence metric of the plurality of first confidence metrics, the respective confidence metric of the plurality of second confidence metrics and the respective confidence metric of the third plurality of confidence metrics.

The inventors have realized that it is particularly advantageous to use three different calculation methods to determine the combined confidence metrics.

In embodiments involving determining a second and a third plurality of confidence metrics, the method may further comprise:
determining each respective confidence metric of the second plurality of confidence metrics comprises determining the degree of similarity between the first acquired image and a respective reference image of the plurality of reference images, and
determining each respective confidence metric of the third plurality of confidence metrics comprises determining the correspondence between anatomical features detected in the first acquired image and each respective reference image of the plurality of reference images.

In particular, the inventors have realized that the comparison between an acquired image and a plurality of reference images can be improved by using information relating to the similarity between an acquired image and a plurality of reference images in combination with the consistency between the anatomical features detected in the images being compared.

In embodiments involving determining combined confidence metric, the method may further comprise:
determining whether the first acquired image matches a respective reference image based on the respective combined confidence metric, and
in response to determining that the first acquired image matches the respective reference image,
labelling the first acquired image as matching the respective reference image.

By basing the labelling of the acquired images on the combined confidence metrics, rather than confidence metrics determined using a single calculation method, the automatic matching process is made more reliable.

The first acquired image may be one of a plurality of acquired images, wherein each of the plurality of acquired images is a medical image acquired using a medical imaging device, and the method may further comprise:
receiving the plurality of acquired images of the patient,
comparing each acquired image of the plurality of acquired images to a plurality of reference images; and
outputting a first plurality of confidence metrics for each acquired image;
wherein the comparing step comprises determining, for each acquired image, a first plurality of confidence metrics using a first calculation method, and
wherein each respective confidence metric of the first plurality of confidence metrics for the acquired image indicates how closely the acquired image matches a respective reference image of the plurality of reference images.

Typically a medical imaging protocol involves acquiring a plurality of medical images. It is advantageous to provide a plurality of confidence metrics for each of the plurality of acquired images. Thereby, it is possible to conveniently compare a stream of images to a plurality of reference images.

In embodiments involving a plurality of acquired images, the method further comprising:
determining whether each acquired image matches a respective reference image, and
in response to determining that an acquired image matches a respective reference image,
labelling the acquired image as matching the respective reference image.

In particular, it is advantageous for each acquired image to be compared to the reference images in order to determine which acquired images match a reference image.

In embodiments involving matching a plurality of acquired images, the method may further comprise:
in response to determining that multiple acquired images match the same reference image,
labelling the acquired image with the confidence metric that indicates the strongest match as the acquired image matching the reference image.

The inventors have realized that it is particular beneficial to provide a method that automatically determines the best match between acquired images and a given reference image. This can help to save time spent processing the images, and reduce the required processing resources.

In embodiments involving matching a plurality of acquired images, the method may further comprise:
displaying each acquired image and the corresponding matching reference image such that the user can view each acquired image and each corresponding matching reference image, and optionally
displaying the respective confidence metric of each acquired image such that the user can view each acquired image, each corresponding matching reference image and each respective confidence metric, and optionally
receiving a user input, and
using the user input to determine whether at least one of the acquired images that has been labelled as matching a reference image should be accepted as a match, rejected as a match or re-labelled to match another reference image.

By displaying the matched acquired images and reference images the user is able to visualize which of the reference images have been captured. This can help guide the user on the completeness of the protocol - i.e., which information still needs to be collected. Thereby enabling the image acquisition time to be optimized.

The inventors have realized that the method can be improved by allowing the user to provide an input to accept/reject or otherwise modify a match. This can increase the accuracy of the matching process.

The method may further comprise,
determining the presence of an abnormality in one of the acquired images;
outputting information indicating that an abnormality has been detected.

By determining and outputting information regarding the presence of an abnormality of an acquired image, the user is provided with useful information that can be used to inform clinical decisions. In particular, the user may decide to stop the protocol if certain abnormalities are detected. This means that it is possible to avoid prolonging protocols unnecessarily or when doing so might compromise patient comfort.

The method may further comprise, the reference images corresponding to the patient being in a rest state, and the first or each acquired image correspond to the patient being in a stress state.

The inventors have realized that this method is particularly useful for comparing images acquired when the patient is at rest with images acquired when the patient is in a stressed state. This can enable a more robust comparison of how specific anatomical structures respond to stress.

The method may further comprise the reference images corresponding to standard anatomical views.

For many applications there are a set of standard views that a user can extract useful clinical information from. Accordingly, it is particularly advantageous if the reference views correspond to these standard views.

The method may further comprise the reference images and the first or each acquired image being cardiac ultrasound images.

One application where this method can be particularly helpful is echocardiography. For example, the reference images may correspond to standard views of the heart when the patient is in the rest state and the acquired images may correspond to the same standard views of heart when the patient is in a stressed state.

The step of calculating the plurality of confidence metrics may include running a prediction algorithm, the prediction algorithm comprising a trained machine learning algorithm, wherein the prediction algorithm is configured to:
receive, as an input, the first or each acquired image, and
determine, as an output, based on the input, the plurality of first, second, third or combined confidence metrics corresponding to the first or each acquired image.

The inventors have realized that machine learning algorithms can be used to accurately determine confidence metrics.

The step of calculating the plurality of first confidence metrics may comprise running a first prediction algorithm, the prediction algorithm comprising a trained machine learning algorithm for calculating the probability that an input image matches a standard anatomical view, wherein the first prediction algorithm is configured to:
receive, as an input, the first or each acquired image, and
determine, as an output, based on the input, the plurality of first confidence metrics corresponding to the first or each acquired image; and optionally
wherein the comparing step further comprises determining a second plurality of confidence metrics using a second calculation method, wherein the second calculation method calculates the confidence metrics in a different way to the first calculation method,
wherein calculating the plurality of second confidence metrics comprises running a second prediction algorithm,
wherein the second prediction algorithm comprises a trained machine learning algorithm for calculating the degree of similarity between two images or for determining the correspondence between anatomical features detected in two images, and
the second prediction algorithm is configured to:
   receive, as an input, the first or each acquired image, and
   determine, as an output, based on the input, the plurality of second confidence metrics corresponding to the first or each acquired image.

In embodiments involving calculating the first plurality of confidence metrics using a first prediction algorithm, and a second plurality of confidence metrics using a second prediction algorithm, the method may further comprise:
calculating a plurality of combined confidence metrics, wherein each respective confidence metric of the plurality of combined confidence metrics indicates how closely the first acquired image matches a respective reference image based on the respective confidence metric of the plurality of first confidence metrics and the respective confidence metric of the plurality of second confidence metrics.

The step of calculating the plurality of first confidence metrics may comprise running a first prediction algorithm, the prediction algorithm comprising a trained machine learning algorithm for calculating the probability that an input image matches a standard anatomical view, and the first prediction algorithm is configured to:
receive, as an input, the first or each acquired image, and
determine, as an output, based on the input, the plurality of first confidence metrics corresponding to the first or each acquired image; and
wherein the comparing step further comprises determining a second plurality of confidence metrics using a second calculation method, wherein the second calculation method calculates the confidence metrics in a different way to the first calculation method,
wherein calculating the plurality of second confidence metrics comprises running a second prediction algorithm,
wherein the second prediction algorithm comprises a trained machine learning algorithm for calculating the degree of similarity between two images, and
the second prediction algorithm is configured to:
   receive, as an input, the first or each acquired image, and
   determine, as an output, based on the input, the plurality of second confidence metrics corresponding to the first or each acquired image; and
   wherein the comparing step further comprises determining a third plurality of confidence metrics using a third calculation method, wherein the third calculation method calculates the confidence metrics in a different way to the first calculation method and the second calculation method,
   wherein calculating the plurality of third confidence metrics comprises running a third prediction algorithm,
   wherein the third prediction algorithm comprises a trained machine learning algorithm for determining the correspondence between anatomical features detected in two images, and
the third prediction algorithm is configured to:
   receive, as an input, the first or each acquired image, and
   determine, as an output, based on the input, the plurality of third confidence metrics corresponding to the first or each acquired image.

In embodiments involving calculating the first plurality of confidence metrics using a first prediction algorithm, a second plurality of confidence metrics using a second prediction algorithm, and a third plurality of confidence metrics using a third prediction algorithm, the method may further comprise:
calculating a plurality of combined confidence metrics, wherein each respective confidence metric of the plurality of combined confidence metrics indicates how closely the first acquired image matches a respective reference image based on the respective confidence metric of the plurality of first confidence metrics, the respective confidence metric of the plurality of second confidence metrics, and the respective confidence metric of the plurality of third confidence metrics.

In embodiments, the method may further comprise:
acquiring the reference images;
labelling the reference images;
wherein labelling of the reference images is performed automatically or with user input.

According to examples in accordance with another aspect of the invention, there is provided a system for implementing the computer implemented method of any method described herein, the system comprising:
a processor configured to carry out the method of any preceding claim.

According to examples in accordance with another aspect of the invention, there is provided a computer program product comprising instructions which, when the program is executed by a computer, cause the computer to carry out the steps of any computer implemented method described herein.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of a computer implemented method of medical imaging, according to an embodiment of the invention.
Fig. 2 shows another example of a computer implemented method of medical imaging, according to an embodiment of the invention.
Fig. 3 shows another example of a computer implemented method of medical imaging, according to an embodiment of the invention.
Fig. 4 shows another example of a computer implemented method of medical imaging, according to an embodiment of the invention.
Fig. 5 shows another example of a computer implemented method of medical imaging, according to an embodiment of the invention.
Fig. 6 shows an example of a display that can be shown as part of computer implemented method of medical imaging according to an embodiment of the invention.
Fig. 7 shows another example of a display that can be shown as part of computer implemented method of medical imaging according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

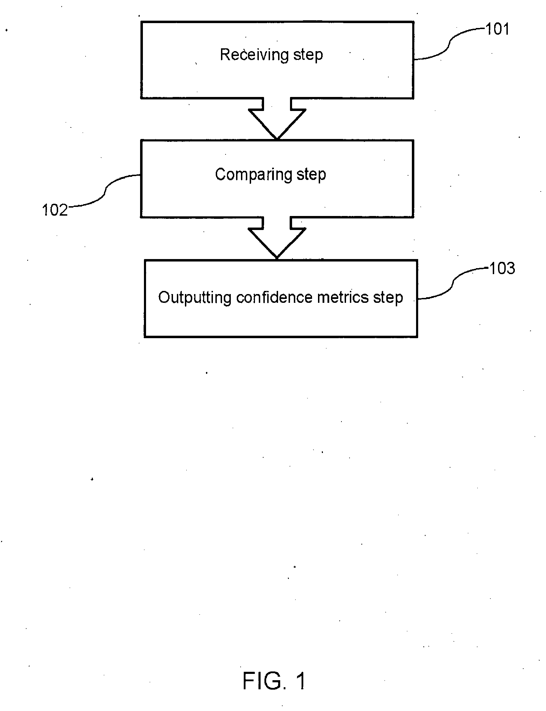

Fig. 1 shows an example of a computer implemented method of medical imaging where acquired images are compared to reference images. The method includes a receiving step 101 wherein at least a first acquired image of a patient is received. In a comparing step 102, the first acquired image is compared to a plurality of reference images. In an outputting confidence metrics step 103, a first plurality of confidence metrics for the first acquired image are outputted. Each respective confidence metric of the first plurality of confidence metrics indicates how closely the first acquired image matches a respective reference image from a plurality of reference images.

In some embodiments, the comparing step involves using a ViewID module. The ViewID module is a deep learning model that is trained in a supervised manner. The deep learning model may be a convolutional neural network (CNN) with several conv-layers, resolutions levels, global average pooling, and a fully-connected output layer. Common architectures such as ResNet, DenseNet, or EfficientNet can be used. The ViewID model can be prepared by training, using a dataset of annotated reference images that are labelled with their respective views. After training, the ViewID model can take an image as its input and output a probability vector that indicates the probability of the current image corresponding to each reference view.

Generally a specific imaging protocol will be associated with a set of standard anatomical views that the user intends to obtain. For stress echo exams, common types of views (i.e. view categories) that the user may want to obtain include various apical views (e.g. AP2, AP3 and/or AP4), short axis (SAX) and parasternal long axis (PLAX).

The output of the ViewID module may be a probability vector where each respective element of the probability vector represents the probability that an acquired image corresponds to the same view as the respective reference image of a plurality of reference images. For example, if there are four reference views labelled V1, V2, V3 and V4, the probability vector for each acquired image with have four elements, where each element corresponds to a specific view. For example, a probability vector [P1, P2, P3, P4] for a first acquired image means that there is a probability P1 that the first acquired image matches the view V1, there is a probability P2 that the first acquired image matches the view V2, there is a probability P3 that the first acquired image matches the view V3 and there is a probability P4 that the first acquired image matches the view V4.

Each probability value contained in the probability vector may represent a binary score (i.e., the acquired image either matches the specific reference image or not). The score may take a value of either 0 or 1. Alternatively, the score may be continuous. For example, each score may take any value between (and including) 0 and 1.

For example, P1 = 0 may mean that there is a 0% probability that the first acquired image matches V1, and P2 = 1 may mean that there is a 100% probability that the first acquired image matches V2.

In embodiments, the reference images may also have corresponding probability vectors, wherein each respective element of the probability vector corresponds to the probability that the reference image corresponds to a respective view. A continuous matching score can be obtained by calculating the cross-entropy, sum of differences, or any other distance measure between the respective probability vectors of a reference image and an acquired image. The resulting score can be normalized to lie between 0 and 1 for easier combination with other similarity measures. The normalization parameters can be obtained by calculating the score for a large number of pairs from a dataset and using the maximum and minimum value of the set of calculated measures.

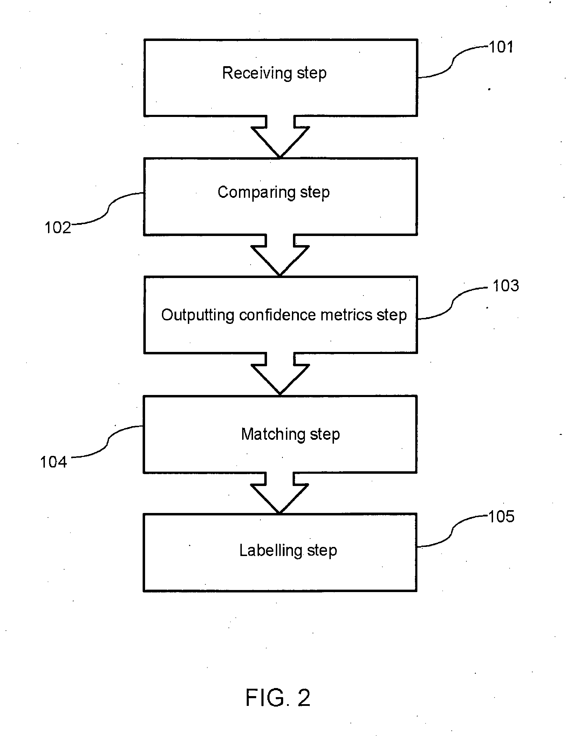

Fig. 2 shows another example of a computer implemented method of medical imaging where acquired images are compared to reference images. This embodiment includes two additional steps compared to the embodiment shown in Fig. 1. The method includes a matching step 104 involving determining whether an acquired image matches a specific reference image. The method also includes a labelling step 105 wherein in response to determining a match between the acquired image and reference image, the acquired image is labelled as matching that reference image.

For example, following a probability vector being obtained from the ViewID module, the method may involve determining whether any of the probabilities indicate that a match between the acquired image and the reference image. This may be assessed by comparing each probability to a threshold value, where any probability over that value indicates a match.

The inventors have realized that the comparison between the acquired images and the reference images can be made more reliable by including further calculation methods in addition to the first calculation method (e.g. the ViewID module). In particular, by using a second calculation method, the confidence metrics can be calculated in a different way compared to the first calculation method (e.g. the probability scoring method used by the ViewID module). The method may further involve calculating a plurality of combined confidence metrics for each acquired image, which takes into account the results from both calculation methods.

In particular, when using the ViewID method, the accuracy of matching may not be as good when the reference view is chosen to be non-standard (e.g. slightly off-angle). The inventors have realized that by using a second calculation method involving a similarity scoring method in combination with the ViewID method the matching accuracy can be improved. The similarity scoring method involves providing a second plurality of confidence metrics, where each respective confidence metric of a second plurality of confidence metrics indicates the degree of similarity between the first acquired image and the respective reference image of the plurality of reference images.

The similarity scoring may be performed using a deep learning model with the purpose of providing similarity scores between an acquired image and a plurality of reference images. A similarity model obtained by a contrastive learning strategy (i.e., a contrastive similarity model) may be used.

The contrastive similarity model uses a trained encoder network, which is trained by contrastive learning. The contrastive similarity model may be trained using pairs of similar and non-similar images. The contrastive similarity model learns to predict a similarity score between the two images. The network may produce a feature vector associated with an input image. During training, the feature vectors of similar images are forced to be close to one another using a distance loss. Dissimilar images are forced to have very different feature vectors. Thus, when two images are fed into a trained contrastive similarity model, their respective feature vectors are generated and compared (e.g. using a cosine similarity distance) to measure how similar they are. To use the contrastive similarity model with a particular imaging modality, the model does not necessarily need to be trained using images acquired via the same imaging modality. For example, to use the contrastive similarity model to compare ultrasound images, the model may be trained using images acquired using other medical imaging modalities, or even natural images. That is, the similarity score may provide a generic indication of the similarity between images. The scores outputted from the similarity model may be normalized to lie between 0 and 1.

Methods for determining the probability that an acquired image shows the same view of the patient as each reference image of a plurality of reference images (e.g. the ViewID model) and methods for calculating the similarity between the acquired and reference images (e.g. the contrastive similarity model) provide very different information. For example, the scores obtained using the ViewID model only provide information about the images' view category. The contrastive method however contains much more abstract information relating to the image similarity, depending on the training images being used. For example, if natural images were used as a training data set, the contrastive similarity model may learn similarity based on features including: edges/borders in the images, object sizes and quantity, or contrast and lighting. If a training data set of ultrasound images showing different regions throughout the body was used, the contrastive similarity model may learn to capture similarity based on the presence of the same organs/anatomical structures in images. If the dataset included images with a variety of pre-sets/filtering/image processing settings then the contrastive similarity model may learn similarity based on the type of image processing applied to the images. Thus, the contrastive method and methods for determining the probability of views matching complement each other as the contrastive method is relatively generic and the methods for determining the probability of views matching are relatively task-specific.

Alternatively, instead of using a similarity scoring method, an anatomy detector model may be used. This involves calculating the second plurality of confidence metrics by determining the correspondence between anatomical features detected in an acquired image and each reference image. For example, the anatomy detector model may be trained to recognize landmarks/anatomical structures such as valves, walls, or the aorta. The anatomy detector model can be deep learning-based. The deep learning model may be an image segmentation model such as a U-Net or a fully-convolutional neural network. This may involve, producing labelled maps of each image. An alternative may be based on the You Only Look Once (YOLO) algorithm for object detection. The model may be trained using images labeled with anatomical bounding box labels.

The presence/absence of certain anatomical structures may be encoded in a categorical way. For example if the reference image contains five anatomical features, and three of those anatomical features are detected in the acquired image, then the score corresponding to the match between the acquired image and the reference image may be 0.6.

A plurality of combined confidence metrics may be calculated based on the plurality of first confidence metrics and the plurality of second confidence metrics. For example, each respective confidence metric of the plurality of combined confidence metrics may be calculated by taking an average, or a weighted average if one of calculation methods is deemed more important to the determination of the plurality of combined confidence metrics.

The inventors have realized that it can be particularly advantageous to use three different calculation methods to determine the combined confidence metrics. For example, the first plurality of confidence metrics may calculated using the ViewID module, the second plurality of confidence metrics may be calculated using contrastive similarity, and a third plurality of confidence metrics may be calculated using an anatomy detector model. The plurality of confidence metrics from each method can be combined to provide a combined plurality of confidence metrics. For example, each respective confidence metric of the plurality of combined confidence metrics may be calculated by taking an average, or a weighted average if one of calculation methods is deemed more important to the determination of the plurality of combined confidence metrics.

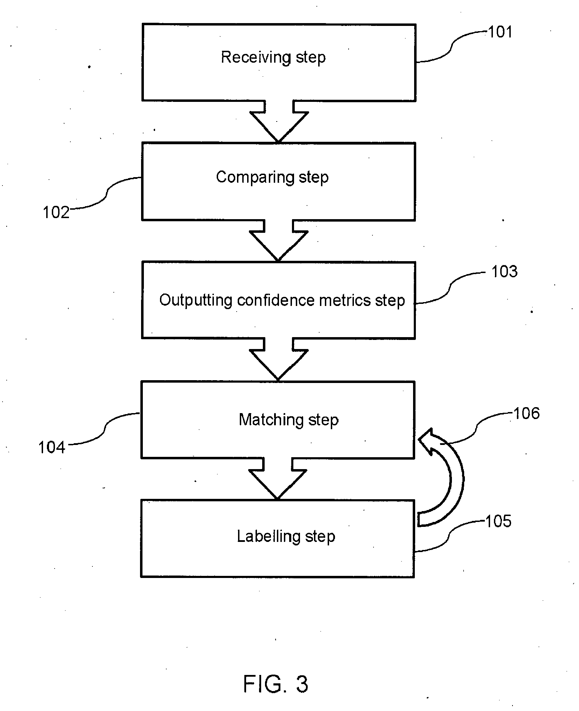

Generally, the first acquired image will be one of a plurality of acquired images. Fig. 3 shows another example of a computer implemented method of medical imaging where acquired images are compared to reference images. This embodiment includes the step 106 which involves, in response to determining that multiple acquired images match the same reference image, labelling the acquired image with the confidence metric that indicates the strongest match as the acquired image matching the reference image.

The plurality of images may be acquired in a continuous stream. Each image of the plurality of images may be processed individually using any of the methods disclosed herein so that the image comparison is provided in real-time. Alternatively, these methods may be performed as a post-processing procedure.

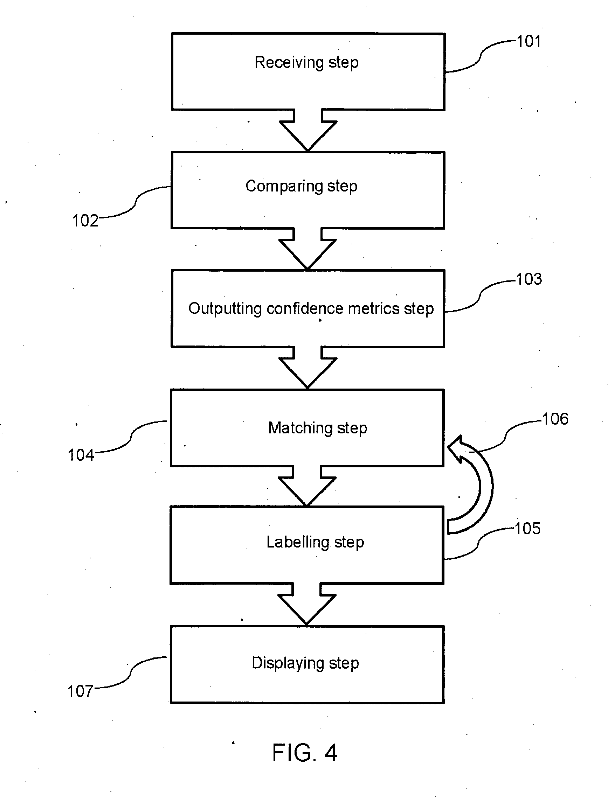

Fig. 4 shows another example of a computer implemented method of medical imaging where acquired images are compared to reference images. This embodiment includes the step 107 where the matched images are displayed. The matched images may be displayed using a graphical user interface (GUI). The GUI may include an indication of whether a matching acquired image has been achieved for each of the reference images. The GUI may also display the score corresponding to each acquired image and the matching reference image. The GUI may also include the functionality to receive a user input. This user input may allow the user to indicate whether each of the acquired images that has been labelled as matching a reference image should be accepted as a match, rejected as a match, or relabeled to match another reference image. That is, the GUI may provide the flexibility of manual image selection by allowing the user to choose more appropriate matches than determined by the calculation methods, if necessary.

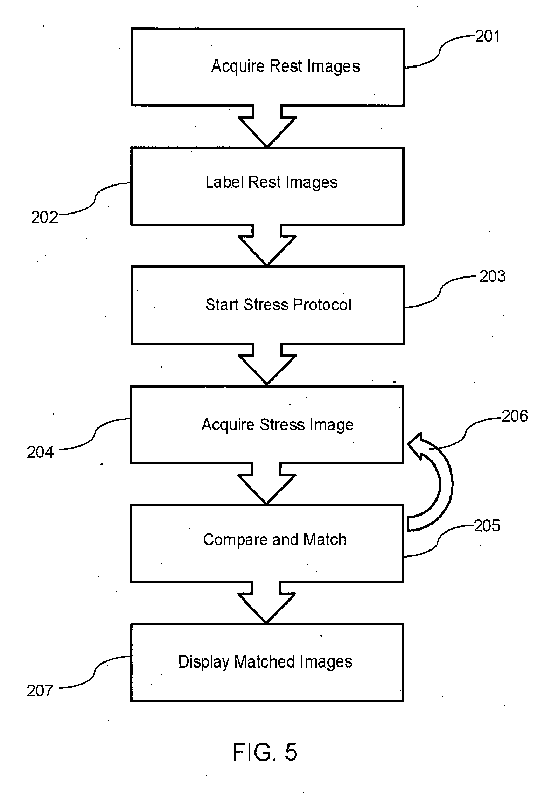

Fig. 5 shows an example workflow including another example of a computer implemented method of medical imaging where acquired images are matched to reference images. In this example, the reference images and the acquired images are cardiac ultrasound images. The reference images correspond to the patient being in a rest state and the acquired images correspond to the patient being in a stress state.

In step 201, the rest images are acquired.

In step 202, these images are labelled. The rest images may be labelled using the ViewID module - although any other method may also be used instead of the ViewID module or in combination with the ViewID module. In some embodiments, user input is taken into account to correct/relabel the rest images.

In step 203, the stress protocol is started - for example by asking the patient to exercise.

In step 204, an image (an "acquired image") corresponding to the patient in a stress state is acquired.

In step 205, the acquired image is compared and matched using at least a first calculation method to provide a first plurality of confidence metrics. Preferably the comparison step involves calculating a plurality of combined confidence metrics using a first plurality of confidence metrics calculated using the ViewID module, a second plurality of confidence metrics calculated using contrastive similarity, and a third plurality of confidence metrics calculated using an anatomy detector method. The confidence metrics may take into account the confidence with which the rest images were labelled.

In step 206, another image may be acquired and matched. This process may be repeated until each rest image has been matched to a stress image.

When a match is determined, the match may be displayed in step 207. The display may be updated after each match is determined.

In an example, the rest and stress images may be matched by determining, by the ViewID module, a probability vector for each rest image and each stress image, wherein the probability vectors indicates the probability that the image matches various standard views. The probability vectors of the rest and stress images can then be compared to match the stress and rest images.

For any of the methods discussed herein, there may be an additional step involving determining whether an acquired image includes a clinical abnormality. The method may involve a step of outputting information indicating that an abnormality has been detected. The determination of whether the acquired image includes an abnormality may be made using a machine learning algorithm. In some embodiments, the method for detecting abnormalities may be performed in parallel with the calculation method used to determine the first plurality of confidence metrics. In other embodiments, only acquired images which have been labelled as matching a reference image will be used as an input to the method for detecting abnormalities. The GUI may include a schematic representation of the anatomy being visualized and indicate on the anatomy where the abnormality has been detected. The GUI may include a recommendation to the user to aid in clinical decisions.

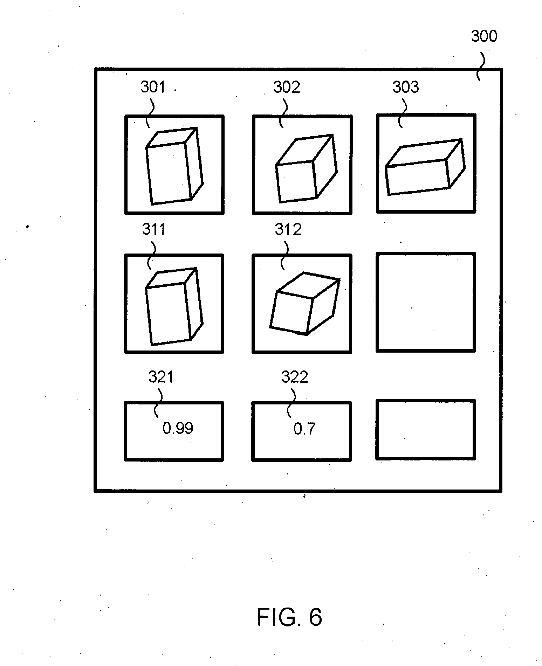

Fig. 6 shows an example of the GUI 300. The GUI may show the reference views 301, 302, 303. The GUI may also show the acquired images 311, 312 that have been matched to a reference view. In addition, the GUI may also include the respective combined confidence metric 321, 322 to provide an indication of the closeness of the respective match. In the example shown in Fig. 6, the GUI shows that a good match has been determined between reference image 301 and the acquired image 311, a less suitable match has been determined between reference image 302 and 312, and no match has yet been found for reference image 303. The GUI may display why a match has been determined as less suitable - for example, because it has been determined as off-angle.

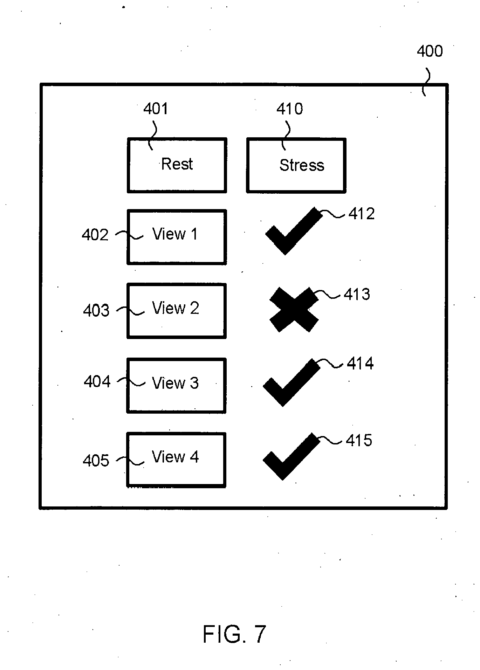

Fig. 7 shows another example of the GUI 400 that can be displayed when performing a method of comparing reference images and stress images. In this example, the reference images and the acquired images are cardiac ultrasound images. The GUI may have two columns, where a first column is labelled as corresponding to rest views 401 and a second column is labelled as corresponding to stress views 410. The first column may display the reference images 402, 403, 404, 405 corresponding to the patient being in the rest state. The second column may display whether a matching image corresponding to the patient being in the stressed state has been acquired 412, 413, 414, 415. In this example, the ticks indicate that a match has been made and a cross indicates that a match has not yet been made. The GUI may also provide the user with an indication that an abnormality has been detected in one of the acquired images.

The inventors have realized that by providing a display where the matches can be shown that the protocol length can be optimized. In particular, it is possible for the user to determine which matches have already been made and which matches are still to be determined. By outputting information on abnormalities that have been automatically detected, the protocol length can be further optimized.

Variations to the disclosed embodiments can be understood and effected by those skilled in the art in practicing the claimed invention, from a study of the drawings, the disclosure and the appended claims.

In embodiments, any number of calculation methods can be used to determine the plurality of combined confidence metrics.

In embodiments, the reference images and acquired images may be two-dimensional or three-dimensional.

In embodiments, any imaging modality may be used to acquire the reference and acquired images. For example, the methods described herein could be used for magnetic resonance imaging or computer tomography.

In embodiments, any of the methods described herein may be transferred to and carried out on a cloud-based platform.

In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa..

Any reference signs in the claims should not be construed as limiting the scope. The application also includes the following embodiments:
[1] A computer implemented method of medical imaging comprising:
   receiving a first acquired image of a patient, wherein the first acquired image is a medical image acquired using a medical imaging device;
   comparing the first acquired image to a plurality of reference images by determining a first plurality of confidence metrics using a first calculation method; and
   outputting the first plurality of confidence metrics for the first acquired image,
   wherein each respective confidence metric of the first plurality of confidence metrics indicates how closely the first acquired image matches a respective reference image of the plurality of reference images.
[2] The computer implemented method of embodiment [1], wherein determining each respective confidence metric of the first plurality of confidence metrics comprises determining the probability that the first acquired image shows the same view of the patient as the respective reference image of the plurality of reference images.
[3] The computer implemented method of embodiment [1] or embodiment [2], further comprising:
   determining whether the first acquired image matches a respective reference image based on the respective confidence metric, and
   in response to determining that the first acquired image matches the respective reference image,
   labelling the first acquired image as matching the respective reference image.
[4] The computer implemented method of embodiment [1] or embodiment [2], wherein the comparing step further comprises:
   determining a second plurality of confidence metrics using a second calculation method, wherein the second calculation method calculates the confidence metrics in a different way to the first calculation method; and
   calculating a plurality of combined confidence metrics, wherein each respective confidence metric of the plurality of combined confidence metrics indicates how closely the first acquired image matches a respective reference image based on the respective confidence metric of the plurality of first confidence metrics and the respective confidence metric of the plurality of second confidence metrics.
[5] The computer implemented method of embodiment [4], wherein determining each respective confidence metric of the second plurality of confidence metrics comprises: determining the degree of similarity between the first acquired image and the respective reference image of the plurality of reference images, or
   determining the correspondence between anatomical features detected in the first acquired image and the respective reference image.
[6] The computer implemented method of either of embodiments [4] or [5], wherein the comparing step further comprises determining a third plurality of confidence metrics using a third calculation method, wherein the third calculation method calculates the confidence metrics in a different way compared to first and second calculation methods, and
   calculating a plurality of combined confidence metrics, wherein each respective combined confidence metric indicates how closely the first acquired image matches a respective reference image based on the respective confidence metric of the plurality of first confidence metrics, the respective confidence metric of the plurality of second confidence metrics and the respective confidence metric of the third plurality of confidence metrics, and optionally wherein
   determining each respective confidence metric of the second plurality of confidence metrics comprises determining the degree of similarity between the first acquired image and a respective reference image of the plurality of reference images, and
   determining each respective confidence metric of the third plurality of confidence metrics comprises determining the correspondence between anatomical features detected in the first acquired image and each respective reference image of the plurality of reference images.
[7] The computer implemented method of any of embodiments [4]-[6],
   further comprising:
   determining whether the first acquired image matches a respective reference image based on the respective combined confidence metric, and
   in response to determining that the first acquired image matches the respective reference image,
   labelling the first acquired image as matching the respective reference image.
[8]. The computer implemented method according to any of embodiments [1]-[7],
   wherein the first acquired image is one of a plurality of acquired images, wherein each of the plurality of acquired images are medical images acquired using a medical imaging device, the method further comprising:
   receiving the plurality of acquired images of the patient,
   comparing each acquired image of the plurality of acquired images to a plurality of reference images; and
   outputting a first plurality of confidence metrics for each acquired image;
   wherein the comparing step comprises determining, for each acquired image, a first plurality of confidence metrics using a first calculation method, and
   wherein each respective confidence metric of the first plurality of confidence metrics for the acquired image indicates how closely the acquired image matches a respective reference image of the plurality of reference images.
[9] The computer implemented method according to embodiment [8], the method further comprising:
   determining whether each acquired image matches a respective reference image, and
   in response to determining that an acquired image matches a respective reference image,
   labelling the acquired image as matching the respective reference image.
[10] The computer implemented method of embodiment [9], further comprising;
   in response to determining that multiple acquired images match the same reference image,
   labelling the acquired image with the confidence metric that indicates the strongest match as the acquired image matching the reference image.
[11] The computer implemented method according either of embodiments [9] or [10], further comprising:
   displaying each acquired image and the corresponding matching reference image such that the user can view each acquired image and each corresponding matching reference image, and optionally
   displaying the respective confidence metric of each acquired image such that the user can view each acquired image, each corresponding matching reference image and each respective confidence metric, and optionally
   receiving a user input, and
   using the user input to determine whether at least one of the acquired images that has been labelled as matching a reference image should be accepted as a match, rejected as a match or re-labelled to match another reference image.
[12] The computer implemented method of any of embodiments [1]-[11] further comprising:
   determining the presence of an abnormality in one of the acquired images; and
   outputting information indicating that an abnormality has been detected.
[13] The computer implemented method of any of embodiments [1]-[12] wherein the reference images correspond to the patient being in a rest state, and the first or each acquired image correspond to the patient being in a stress state.
[14] The computer implemented method of any of embodiments [1]-[13] wherein the reference images correspond to standard anatomical views, and/or
   wherein the reference images and the first or each acquired image are cardiac ultrasound images.
[15] The computer implemented method of any of embodiments [1]-[14] wherein calculating the plurality of first confidence metrics comprises running a first prediction algorithm, the first prediction algorithm comprising a trained machine learning algorithm for calculating the probability that an input image matches a standard anatomical view, and the first prediction algorithm is configured to:
   receive, as an input, the first or each acquired image, and
   determine, as an output, based on the input, the plurality of first confidence metrics corresponding to the first or each acquired image; and optionally
   wherein the comparing step further comprises determining a second plurality of confidence metrics using a second calculation method, wherein the second calculation method calculates the confidence metrics in a different way to the first calculation method,
   wherein calculating the plurality of second confidence metrics comprises running a second prediction algorithm,
   wherein the second prediction algorithm comprises a trained machine learning algorithm for calculating the degree of similarity between two images or for determining the correspondence between anatomical features detected in two images, and
   the second prediction algorithm is configured to:
      receive, as an input, the first or each acquired image, and
      determine, as an output, based on the input, the plurality of second confidence metrics corresponding to the first or each acquired image.
[16] A system for implementing the computer implemented method of any of embodiments [1]-[15], the system comprising:
   a processor configured to carry out the method of any preceding embodiment.
[17] A computer program product comprising instructions which, when the program is executed by a computer, cause the computer to carry out the steps of the computer implemented method of any of embodiments [1]-[15].

## Claims

1. A computer implemented method of medical imaging comprising:
receiving a first acquired image of a patient, wherein the first acquired image is a medical image acquired using a medical imaging device;
comparing the first acquired image to a plurality of reference images by determining a first plurality of confidence metrics using a first calculation method; and
outputting the first plurality of confidence metrics for the first acquired image,
wherein each respective confidence metric of the first plurality of confidence metrics indicates how closely the first acquired image matches a respective reference image of the plurality of reference images.

2. The computer implemented method of claim 1, wherein determining each respective confidence metric of the first plurality of confidence metrics comprises determining the probability that the first acquired image shows the same view of the patient as the respective reference image of the plurality of reference images.

3. The computer implemented method of claim 1 or claim 2, further comprising:
determining whether the first acquired image matches a respective reference image based on the respective confidence metric, and
in response to determining that the first acquired image matches the respective reference image,
labelling the first acquired image as matching the respective reference image.

4. The computer implemented method of claim 1 or claim 2, wherein the comparing step further comprises:
determining a second plurality of confidence metrics using a second calculation method, wherein the second calculation method calculates the confidence metrics in a different way to the first calculation method; and
calculating a plurality of combined confidence metrics, wherein each respective confidence metric of the plurality of combined confidence metrics indicates how closely the first acquired image matches a respective reference image based on the respective confidence metric of the plurality of first confidence metrics and the respective confidence metric of the plurality of second confidence metrics.

5. The computer implemented method of claim 4, wherein determining each respective confidence metric of the second plurality of confidence metrics comprises:
determining the degree of similarity between the first acquired image and the respective reference image of the plurality of reference images, or
determining the correspondence between anatomical features detected in the first acquired image and the respective reference image.

6. The computer implemented method of either of claims 4 or 5, wherein the comparing step further comprises determining a third plurality of confidence metrics using a third calculation method, wherein the third calculation method calculates the confidence metrics in a different way compared to first and second calculation methods, and
calculating a plurality of combined confidence metrics, wherein each respective combined confidence metric indicates how closely the first acquired image matches a respective reference image based on the respective confidence metric of the plurality of first confidence metrics, the respective confidence metric of the plurality of second confidence metrics and the respective confidence metric of the third plurality of confidence metrics, and optionally wherein
determining each respective confidence metric of the second plurality of confidence metrics comprises determining the degree of similarity between the first acquired image and a respective reference image of the plurality of reference images, and
determining each respective confidence metric of the third plurality of confidence metrics comprises determining the correspondence between anatomical features detected in the first acquired image and each respective reference image of the plurality of reference images.

7. The computer implemented method of any of claims 4-6,
further comprising:
determining whether the first acquired image matches a respective reference image based on the respective combined confidence metric, and
in response to determining that the first acquired image matches the respective reference image,
labelling the first acquired image as matching the respective reference image.

8. The computer implemented method according to any of claims 1-7,
wherein the first acquired image is one of a plurality of acquired images, wherein each of the plurality of acquired images are medical images acquired using a medical imaging device, the method further comprising:
receiving the plurality of acquired images of the patient,
comparing each acquired image of the plurality of acquired images to a plurality of reference images; and
outputting a first plurality of confidence metrics for each acquired image;
wherein the comparing step comprises determining, for each acquired image, a first plurality of confidence metrics using a first calculation method, and
wherein each respective confidence metric of the first plurality of confidence metrics for the acquired image indicates how closely the acquired image matches a respective reference image of the plurality of reference images.

9. The computer implemented method according to claim 8, the method further comprising:
determining whether each acquired image matches a respective reference image, and
in response to determining that an acquired image matches a respective reference image, labelling the acquired image as matching the respective reference image.

10. The computer implemented method according to claim 9, further comprising:
displaying each acquired image and the corresponding matching reference image such that the user can view each acquired image and each corresponding matching reference image, and optionally
displaying the respective confidence metric of each acquired image such that the user can view each acquired image, each corresponding matching reference image and each respective confidence metric, and optionally
receiving a user input, and
using the user input to determine whether at least one of the acquired images that has been labelled as matching a reference image should be accepted as a match, rejected as a match or re-labelled to match another reference image.

11. The computer implemented method of any preceding claim wherein the reference images correspond to the patient being in a rest state, and the first or each acquired image correspond to the patient being in a stress state.

12. The computer implemented method of any preceding claim wherein the reference images correspond to standard anatomical views, and/or
wherein the reference images and the first or each acquired image are cardiac ultrasound images.

13. The computer implemented method of any preceding claim wherein calculating the plurality of first confidence metrics comprises running a first prediction algorithm, the first prediction algorithm comprising a trained machine learning algorithm for calculating the probability that an input image matches a standard anatomical view, and the first prediction algorithm is configured to:
receive, as an input, the first or each acquired image, and
determine, as an output, based on the input, the plurality of first confidence metrics corresponding to the first or each acquired image; and optionally
wherein the comparing step further comprises determining a second plurality of confidence metrics using a second calculation method, wherein the second calculation method calculates the confidence metrics in a different way to the first calculation method,
wherein calculating the plurality of second confidence metrics comprises running a second prediction algorithm,
wherein the second prediction algorithm comprises a trained machine learning algorithm for calculating the degree of similarity between two images or for determining the correspondence between anatomical features detected in two images, and
the second prediction algorithm is configured to:
receive, as an input, the first or each acquired image, and
determine, as an output, based on the input, the plurality of second confidence metrics corresponding to the first or each acquired image.

14. A system for implementing the computer implemented method of any preceding claim, the system comprising:
a processor configured to carry out the method of any preceding claim.

15. A computer program product comprising instructions which, when the program is executed by a computer, cause the computer to carry out the steps of the computer implemented method of any of claims 1-13.
